(19) Europäisches Patentamt
European Patent Office
Office européen des brevets

(11) Publication number: **0 139 809**
**B1**

(12) # EUROPEAN PATENT SPECIFICATION

(45) Date of publication of patent specification: **27.07.88**

(21) Application number: **84100466.6**

(22) Date of filing: **18.01.84**

(51) Int. Cl.⁴: **C 07 D 311/22,** C 07 D 311/24, C 07 D 311/58, C 07 D 405/06

(54) **Substituted dihydrobenzopyrans.**

(30) Priority: **08.08.83 US 520973**
**12.12.83 US 560355**

(43) Date of publication of application:
**08.05.85 Bulletin 85/19**

(45) Publication of the grant of the patent:
**27.07.88 Bulletin 88/30**

(84) Designated Contracting States:
**BE CH DE FR GB IT LI NL SE**

(56) References cited:
**EP-A-0 079 637**
**EP-A-0 129 906**
**GB-A-2 089 338**
**US-A-4 234 726**
**US-A-4 281 008**

**CHEMICAL ABSTRACTS, vol. 97, no. 3, July 19, 1982, Columbus, Ohio, USA SHEARD, P.; HOLROYDE, M.C.; GHELANI, A.M.; BANTICK, J.R.; LEE, T.B. "Antagonists of SRS-A and leukotrienes" page 44, column 1, abstract-no. 16 807g**

(73) Proprietor: **G.D. Searle & Co.**
**P.O. Box 1045**
**Skokie Illinois 60076 (US)**

(72) Inventor: **Miyano, Masateru**
**2139 Valley Road**
**Northbrook Illinois 60062 (US)**
Inventor: **Shone, Robert L.**
**1441 Joan Drive**
**Palatine Illinois 60067 (US)**
Inventor: **Sohn, Daniel D.**
**c/o Lundgren Jakosbergs Platsen 6A**
**S-724 61 Vuasteris (SE)**

(74) Representative: **Wolff, Hans Joachim, Dr.jur. Dipl.-Chem. et al**
**Beil, Wolff & Beil Rechtsanwälte Postfach 80 01 40 Adelonstrasse 58**
**D-6230 Frankfurt am Main 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 139 809 B1**

(56) References cited:

CHEMICAL ABSTRACTS, vol. 98, no. 15, April 11, 1983, Columbus, Ohio, USA CASEY, F.B.; APPLEBY, B.J.; BUCK, D.C. "Selective inhibition of the lipoxygenase metabolic pathway of arachidonic acid by the SRS-A antagonist, FPL 55712" page 152, column 2, abstract-no. 120 364s

CHEMICAL ABSTRACTS, vol. 98, no. 23, June 6, 1983, Columbus, Ohio, USA WELTON, A.F.; O'DONNELL, M.; ANDERSON, W.; CROWLEY, H.; MEDFORD, A.; SIMKO, B.; YAREMKO, B. "Role of cyclooxygenase products in some of the biological effects of chemically synthesized leukotrienes (B4, C4, D4 and E4)" page 141, column 2, abstract-no. 192 391w

CHEMICAL ABSTRACTS, vol. 98, no. 7, February 14, 1983, Columbus, Ohio, USA FENIUK, LINDA; KENNEDY, I.; WHELAN, C.J. "The contractile action of slow reacting substance of anaphylaxis (SRS-A) on rat isolated fundic strip and guinea pig isolated ileum and its antagonism by FPL 55712" page 148, column 1, abstract-no. 47606r

**Description**

BACKGROUND OF THE INVENTION

a) Field of the Invention

This invention in its broadest aspect, relates to metabolic inhibitors. In particular the invention relates to novel compounds of Formula I which are inhibitors of leukotriene $D_4$ ($LTD_4$) and therefore useful to prevent or alleviate the symptoms or conditions associated with $LTD_4$ such as allergic reactions and inflammatory conditions.

$LTD_4$ is a product of the 5-lipoxygenase pathway and is the major active constituent of slow reacting substance of anaphylaxis (SRS-A) in humans and guinea pigs, Lewis *et al.*, Nature USA, *293*: 103—108, (1981). It is a potent bronchoconstrictor that is released during allergic reactions. Because antihistamines are ineffective in the management of asthma it is believed that SRS-A mediates bronchoconstriction resulting from an allergic attack. SRS-A is also a potent inducer of vascular permiability, and it also may be involved in other inflammatory conditions such as rheumatoid arthritis, Geller, J., *et al.*, J. Clin. Endocrinol. Metab. *43*: 686—688, (1976).

b) Information Disclosure

Appleton *et al.,* J. Med. Chem. *20*, 371—379 (1977) discloses a series of chromone-2-carboxylic acids which are antagonists of SRS-A. Specifically sodium 7-[3-(4-acetyl-3-hydroxy-2-propylphenoxy)-2-hydroxypropoxy]-4-oxo-8-propyl-4H-1-benzopyran-2-carboxylate (FPL 55712), appears to be the first reported specific antagonist of SRS-A and $LTD_4$. EP—A—0 079 637 discloses anti-SRS-A carboxylic acid derivatives some of which being structurally similar to those of the claimed compounds.

EP—A—O 129 906 which is an older application according to Article 54(3) EPC discloses phenoxyalkoxy-3,4-dihydro-2H-1-benzopyran derivatives which differ in some respects from the claimed substituted dihydrobenzopyrans.

SUMMARY OF THE INVENTION

The invention relates to compounds of the formula:

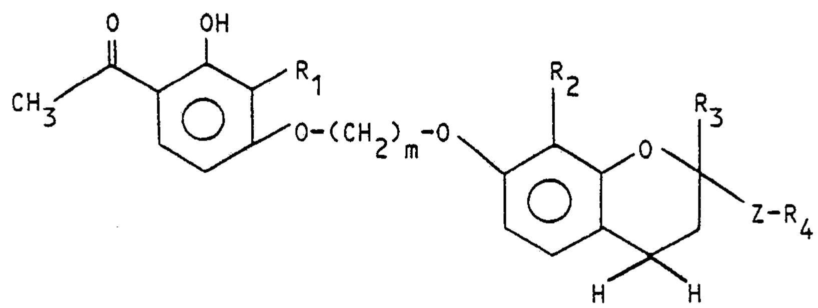

wherein Z is:
- a) $-(CH_2)_n-$; or
- b) $-(CH_2)_p-CH=CH-(CH_2)_q-$;

wherein m is an integer of from 2 to 6 inclusive;
wherein n is an integer of from 1 to 3 inclusive;
wherein p is an integer of from zero to 4 inclusive;
wherein q is an integer of from zero to 4 inclusive;
wherein p + q is equal to or less than 6;
wherein $R_1$, $R_2$ and $R_6$ are:
- a) alkyl of 1 to 6 carbon atoms, inclusive, each being the same or different;

wherein $R_3$ is:
- a) hydrogen; or
- b) alkyl of 1 to 6 carbon atoms, inclusive;

wherein $R_4$ is:
- a) $-CO_2H$;
- b) $-CO_2R_6$;
- c) $-CONR_7R_8$; or
- d) $-OH$

wherein $R_7$ and $R_8$ are:
- a) hydrogen;
- b) alkyl of 1 to 6 carbon atoms, inclusive; $R_7$ and $R_8$ each being the same or different; or
- c) taken together with the nitrogen atom to which they are attached form a 5 or 6 member saturated N-heterocycle optionally substituted by carboxylic acid or alkyl esters thereof of 1 to 6 carbon atoms, inclusive, with the balance of the members being carbon;

or the pharmacologically acceptable addition salts thereof.

Examples of alkyl of 1 to 6 carbon atoms inclusive are methyl, ethyl, propyl, butyl, pentyl, hexyl, and the isomeric forms thereof.

Salts of the acid forms of these compounds can be prepared by neutralization with the appropriate amount of an inorganic or organic base such as sodium hydroxide, potassium hydroxide, calcium hydroxide, magnesium hydroxide, ammonia, trialkylamine, dialkylamine, monoalkylamine, dibasic amino acids, sodium acetate, potassium benzoate, triethanolamine and like bases.

$LTD_4$ causes bronchoconstriction when administered to humans and guinea pigs. The bronchoconstriction has 2 components: a) a direct stimulation of respiratory smooth muscle by $LTD_4$ and b) an indirect component through release of thromboxane A2 which in turn causes contraction of respiratory smooth muscle. Compounds of the invention antagonize the direct component. The compounds are tested *in vivo* as follows.

Adult male fasted Hartly guinea pigs weighing 300—350 g are pretreated with pyrilamine and indomethacin to block the bronchoconstricture effects of endogenous histamine and the synthesis of thromboxane A2 respectively. Compounds of the invention are administered IV or IG at appropriate times prior to the IV administration of 2000 units of $LTD_4$. Intratracheal pressure is monitored prior to and subsequent to $LTD_4$ in animals anesthetized with pentobarbital and attached to a rodent respirator. Compounds which antagonize the direct component of $LTD_4$ action on respiratory smooth muscle inhibit intratracheal insufflation pressure increases (P or =0.05) caused by $LTD_4$. FPL 55712 is used as a control.

The compounds can be administered in a number of dosage forms. A preferred method of delivery would be oral or in such a manner so as to localize the action of the inhibitor. For example, for asthma, the compounds could be inhaled using an aerosol or other appropriate spray. In an inflammatory condition such as rheumatoid arthritis the compounds could be injected directly into the affected joint. The compounds could also be administered in oral unit dosage forms such as tablets, capsules, pills, powders or granules. They also may be administered rectally or vaginally in such forms as suppositories. They may be introduced in the form of eyedrops, intraperitoneally, subcutaneously, or intramuscularly using forms known to the pharmaceutical art. For the treatment of inflammatory allergic skin conditions, the compounds of the present invention may also be administered topically in the form of ointments, creams, gels or the like. Regardless of the route of administration selected, the compounds are formulated into pharmaceutically acceptable dosage forms by conventional methods known to the pharmaceutical art.

An effective but non-toxic quantity of the compound is employed in treatment. The dosage regimen for inhibition of $LTD_4$ by the compounds of this invention is selected in accordance with a variety of factors including the type, age, weight, sex, and medical condition of the mammal, the particular disease and its severity, the route of administration and the particular compound employed. An ordinarily skilled physician or veterinarian will readily determine and prescribe the effective amount of the compound to prevent or arrest the progress of the condition. In so proceeding, the physician or veterinarian could employ relatively low dosages at first, subsequently increasing the dose until a maximum response is obtained.

The compounds of this invention are prepared by the general methods illustrated in Charts A to E. The various compounds and intermediates can be readily modified by methods known to those skilled in the art. For example, esters can be hydrolyzed to corresponding carboxylic acids (and their respective addition salts), converted to corresponding amides by appropriate reactions with amines, and reduced to alcohols by such reagents as Lithium borohydride. Such products and intermediates can, of course, be similarly interconverted.

As illustrated in Chart A, 2-hydroxyacetophenones of Formula XI react readily with ketones of Formula XII to afford fused ring compounds of Formula XIII. Ketones, Formula XII, include ketoesters in which Z is alkylene or alkenylene and $R_4$ is alkoxycarbonyl. An example of such a ketoester is ethyl levulinate. Preferred condensation and cyclization conditions include heating Formulas XI and XII at reflux in toluene, in the presence of a base such as pyrrolidine, with provisions for the removal of water with an apparatus such as a Dean-Stark trap. Intermediates of Formula XIII thus formed may be used in reactions of Chart B without further modification or they may be converted to related intermediates, Formula XIV, by methods known to those skilled in the art. For example, hydrogenation over palladium on carbon will reduce the keto function of Formula XIII to the corresponding dihydrobenzopyran, Formula XIV ($Y=H_2$).

As illustrated in Chart B, compounds of Formula XIV may be alkylated under basic conditions to form compounds of Formula XXI. Preferred reagents include dihaloalkanes, such as 1,3-dibromopropane and the like. Preferred conditions include reaction in dry dimethylformamide in the presence of anhydrous potassium carbonate. Intermediates XXI are typically purified by column chromatography on silica gel. Reaction of these intermediates with 2-hydroxyacetophenones of Formula XXII afford title compounds of this invention, Formula XXIII. Preferred conditions include reaction in dry dimethylformamide in the presence of anhydrous potassium carbonate. Alternatively, the reaction may be run under phase transfer conditions.

Chart C illustrates preparation of compounds XXIII using a variation of the method of Chart B. 2-Hydroxyacetophenones of Formula XXII react with dihaloalkanes as described above (See Chart B) to form intermediates of Formula XXXI. By the same general procedure employed in converting Formula XIV to Formula XXI, compounds XXXI and XIV react to form the title compounds of this invention, Formula XXIII.

Chart D illustrates another route for preparing some of the compounds of this invention. Benzopyrones

of Formula XLI, previously described in the literature, can be reduced by catalytic hydrogenation to compounds of Formula XLII.

Esters ($R_4$=COO(alkyl) can be reduced with active metal hydrides, such as Lithium borohydride, to give corresponding alcohols of Formula XLIV.

Chart E also illustrates another approach to preparing some of the compounds of this invention. Compounds of Formula LI (in which $R_{11}$ may be a protecting group to be removed for later elaboration or may consist of the ultimate aromatic side chain already prepared as generally described above) are first treated with strong base in an unreactive solvent. Preferred reagents include n-butyl lithium in tetrahydrofuran. Alkylation affords compounds of Formula LII. Examples of alkylating reagents include simple alkyl halides or substituted alkyl halides, such as iodoalkylcarboxylic ester, beta-unsaturated carboxylic esters (which undergo conjugate additions), and many other reagents known to those skilled in the art. Further conversions of compounds LII can produce other derivatives, such as free carboxylic acids, Formula LIII, which can be formed by hydrolysis of compounds LII. Acylation of compounds LI under similar conditions afford compounds of Formula LIV. Acylating reagents include acyl halides or other activated acyl derivatives. Preferred reagents include substituted N-alkanoylimidazole derivatives. Compounds of Formula LIV can also be converted to other derivatives. For example, basic hydrolysis leads to decarboxylated compounds of Formula LV.

## DESCRIPTION OF THE PREFERRED EMBODIMENTS

In the following Examples intermediates are defined as "A" and products of the invention as "B".

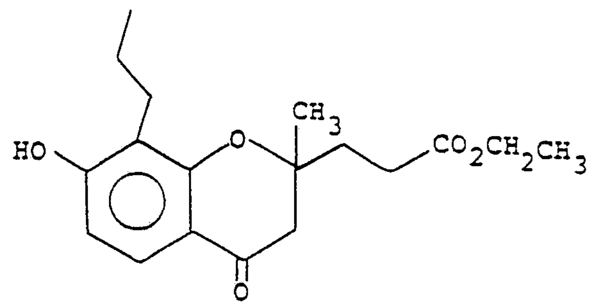

(A)

### Example 1

ethyl 3-(3,4-dihydro-7-hydroxy-2-methyl-4-oxo-8-propyl-2H-1-benzopyran-2-yl)propanoate

A solution of 58.0 g (0.299 mole) of 2,4-dihydroxy-3-propylacetophenone and 29.5 ml (0.36 mole) of pyrrolidine in 250 ml of toluene was heated to reflux for three hours under a Dean-Stark trap. After cooling the mixture, 68.2 ml (0.48 mole) of ethyl levulinate was added and the mixture was refluxed for two hours. An additional 10 ml (0.12 mole) of pyrrolidine was added and the mixture was refluxed overnight under a Dean-Stark trap.

The reaction mixture was diluted with ethyl acetate and washed successively with water, 2 *M* hydrochloric acid, water and brine. It was then dried over $MgSO_4$, filtered, and evaporated to dryness. The residue was chromatographed on silica gel using 30% ethyl acetate/hexane as eluent to give 35.5 g of the title ester, mp. 92—94°. Structure assignment was confirmed by nmr and infrared spectra.

IR ($CHCl_3$): 1665, 1730 $cm^{-1}$.

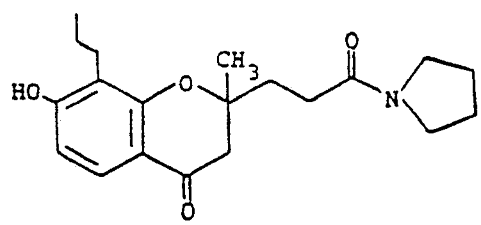

(A)

### Example 2

1-[3-[3,4-dihydro-7-hydroxy-2-methyl-4-oxo-8-propyl-2H-1-benzopyran-2-yl)-1-oxopropyl]pyrrolidine

Other chromatographic fractions of Example 1 afforded 30 g of the title amide, m.p. 182—184°. Structure assignment was confirmed by nmr and infrared spectra and by elemental analysis.

IR (KBr): 1615, 1675 $cm^{-1}$.

| | | | |
|---|---|---|---|
| Analysis. Calcd. for $C_{20}H_{27}NO_4$: | C, 69.54; | H, 7.88; | N, 4.05. |
| Found: | C, 69.22; | H, 7.93; | N, 3.97. |

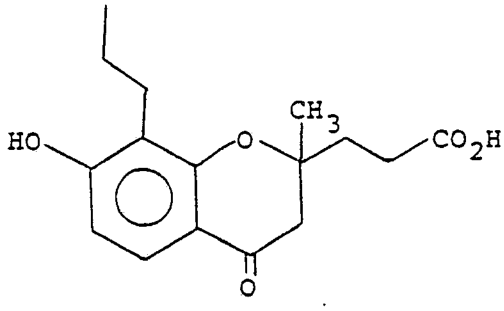

(A)

Example 3

3-(3,4-dihydro-7-hydroxy-2-methyl-4-oxo-8-propyl-2H-1-benzopyran-2-yl)propanoic acid

To a mixture of 20.0 g (103 mmole) of 2,4-dihydroxy-3-propylacetophenone and 15.5 g (134 mmole) of methyl levulinate in 100 ml of dry toluene was added by syringe 21.5 ml (*ca.* 260 mmole) of pyrrolidine. After stirring for one hour the solution was heated on a steam bath for four hours and allowed to cool. The toluene solution was washed with water and 2 *N* NaOH. The basic solution was acidified and extracted with diethyl ether, which in turn was extracted with saturated sodium bicarbonate. The basic aqueous extract was neutralized with dilute hydrochloric acid and again extracted with diethyl ether. After drying, the solution was concentrated at reduced pressure to an oil which crystallized to the title compound, m.p. 152—153°.

| | | |
|---|---|---|
| Analysis calcd. for $C_{16}H_{20}O_5$: | C, 65.74; | H, 6.90. |
| Found: | C, 65.24; | H, 6.86. |

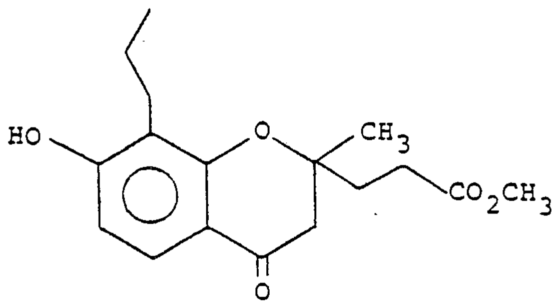

(A)

Example 4

methyl 3-(3,4-dihydro-7-hydroxy-2-methyl-4-oxo-8-propyl-2H-1-benzopyran-2-yl)propanoate

A mixture of the title compound of Example 3 (4.3 g, 14.7 mmole), 6 ml of trimethylorthoformate, and 1.4 ml of sulfuric acid was stirred at room temperature for two hours. The reaction mixture was poured onto stirred ice/water and extracted with ethyl acetate. The organic phase was washed with water, dried over magnesium sulfate, and concentrated to an oil. The oil was purified by chromatography on silica gel, giving 3.7 g of the title compound as a crystalline solid, m.p. 101.5—102.5°. Structure assignment was consistent with nmr and infrared spectra.

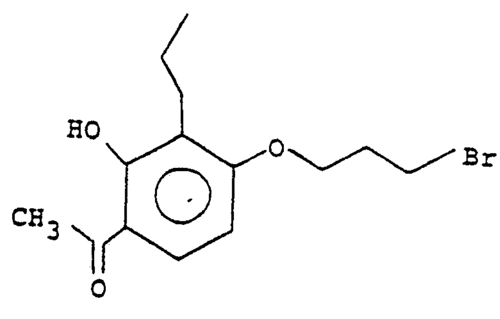

(A)

Example 5

4-(3-bromopropoxy)-2-hydroxy-3-propylacetophenone

To a mixture of 50.0 g (257 mmole) of 2,4-dihydroxy-3-propylacetophenone, 87.4 g (257 mmole) of tetrabutylammonium hydrogen sulfate, and 52.2 ml (*ca.* 514 mmole) of dibromopropane in 250 ml of dichloromethane was added 225 ml (450 mmole) of 2 *N* NaOH. The mixture was then heated at reflux for about 30 min. and allowed to cool. After concentrating the reaction mixture under vacuum, the residue was triturated with diethyl ether, and the ether solution was filtered and concentrated to dryness. After purification by column chromatography on silica gel, the title compound was isolated and used in subsequent reactions without further characterization.

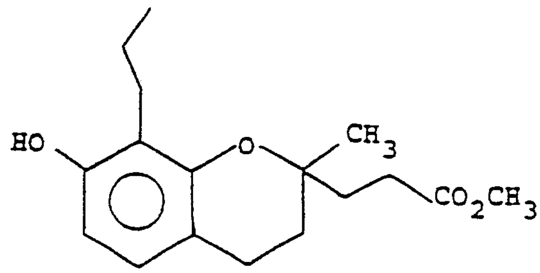

(A)

Example 6

methyl 3-(3,4-dihydro-7-hydroxy-2-methyl-8-propyl-2H-1-benzopyran-2-yl)propanoate

A solution of 4.1 g (13.4 mole) of ester product of Example 4 in 100 ml of acetic acid and 50 ml of methanol was hydrogenated for 21 hours at 4 psi and room temperature using palladium on carbon as catalyst. The catalyst was filtered and the filtrate was evaporated to dryness. Chromatography of the residue on silica gel using 20% ethyl acetate/hexane as eluent gave 1.8 g of the title compound as an oil. Structure assignment was confirmed by nmr and infrared spectra.

nmr ($CKCl_3$): δ (ppm) 0.94 (t, J=7Hz, 3H, propyl $CH_3$); 1.23 (s, 3H, 2-methyl protons); 3.67 (s, 3H, ester $CH_3$); 6.28 (d, J=8Hz, 1H, aromatic); 6.71 (d, J=8Hz, 1H, aromatic).

IR ($CHCl_3$): 1730 $cm^{-1}$.

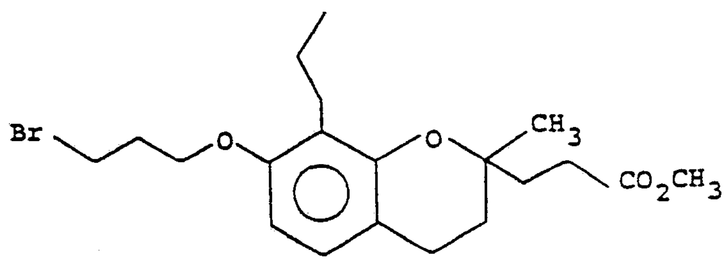

(A)

Example 7

methyl 3-[7-(3-bromopropoxy)-3,4-dihydro-2-methyl-8-propyl-2H-1-benzopyran-2-yl]propanoate

To a solution of 1.7 g (5.51 mmole) of the title compound from Example 6, 1.1 ml (11 mmoles) of 1,3-dibromopropane and 1.9 g (11 mmole) of tetrabutylammonium hydrogen sulfate in 12 ml of methylene chloride was added 5.5 ml of 2 *M* sodium hydroxide solution. The reaction mixture was heated to reflux for 15 minutes and cooled. The organic layer was separated, washed with brine, dried over sodium sulfate, filtered, and concentrated to dryness. Chromatography of the residue on silica gel using 10% ethyl acetate/hexane as eluent gave 1.8 g of the title compound as an oil. Structure assignment was confirmed by nmr and infrared spectra.

nmr ($CDCl_3$): addition of $CH_2Br$ signal at δ (ppm) 4.02 (t, J=6Hz) (compare Example 6).

IR ($CHCl_3$): 1735 $cm^{-1}$.

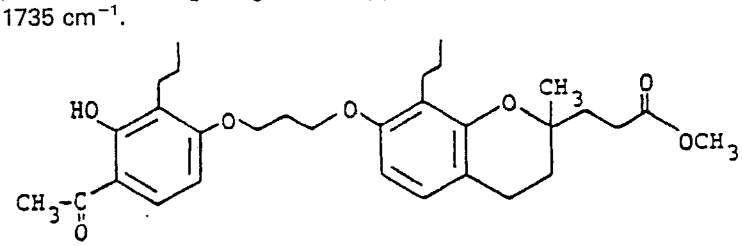

(B)

Example 8

methyl 3-[7-[3-(4-acetyl-3-hydroxy-2-propylphenoxy)propoxy]-3,4-dihydro-2-methyl-8-propyl-2H-1-benzopyran-2-yl]propanoate

A mixture of 6.2 mmole of the ester product of Example 7, 6.8 mmole of 2,4-dihydroxy-3-propylacetophenone and 1.8 g (13 mmole) of anhydrous potassium carbonate in 30 ml of dry dimethylformamide was stirred for 16 hours at room temperature. The inorganic salts were removed by filtration and the dimethylformamide was evaporated *in vacuo*. The residue was dissolved in ethyl acetate and additional inorganic salts were filtered. The filtrate was evaporated and the residue was chromatographed on silica gel using 7% ethyl acetate/toluene as eluent to afford the title compound mp 53—55°C.

Structure assignment was confirmed by nmr and infrared spectra and by elemental analysis.

IR ($CHCl_3$): 1625, 1730 $cm^{-1}$.

| | | |
|---|---|---|
| Analysis. Calcd. for $C_{31}H_{42}O_7$: | C, 70.70; | H, 8.04. |
| Found: | C, 70.59; | H, 8.10. |

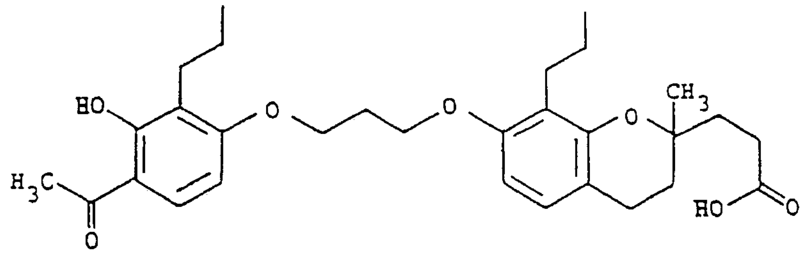

(B)

Example 9

3-[7-[3-(4-acetyl-3-hydroxy-2-propylphenoxy)propoxy]-3,4-dihydro-2-methyl-8-propyl-2H-1-benzopyran-2-yl]propanoic acid

A solution of 0.5 g (19.5 mmole) of lithium hydroxide in 10 ml of water was added to a solution of 3.9 mmole of the title compound of Example 8 in 25 ml of tetrahydrofuran and 20 ml of methanol. After the reaction mixture was stirred for 3 hours at room temperature, the solvent was evaporated and water was added to the residue. After extracting with a small amount of diethyl ether, the aqueous layer was acidified with dilute hydrochloric acid and extracted with ethyl acetate. The organic layer was then washed with water and brine, dried over $MgSO_4$, filtered, and evaporated to dryness to afford the title compound as an oil. Structure assignment was confirmed by nmr and infrared spectra and by elemental analysis.

IR (KBr): 1620, 1705, 1735 $cm^{-1}$.

| | | |
|---|---|---|
| Analysis. Calcd. for $C_{30}H_{40}O_7$: | C, 70.29; | H, 7.87. |
| Found: | C, 69.93; | H, 7.77. |

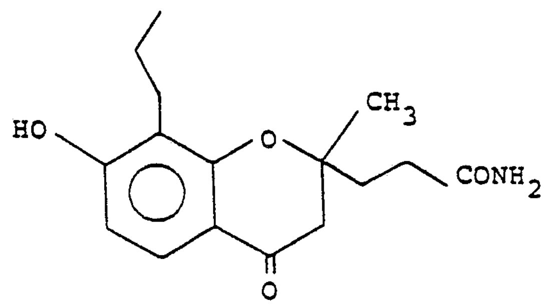

(A)

Example 10

3-(3,4-dihydro-7-hydroxy-2-methyl-4-oxo-8-propyl-2H-1-benzopyran-2-yl)propanamide

To a solution of the title product of Example 3, (1.0 g, 3.4 mmole) in 50 ml of dry tetrahydrofuran cooled to 0° was added 2.1 g (10.2 mmole) of phosphorus pentachloride. After the mixture was stirred for 30 minutes, approximately 2 ml of liquid ammonia was added, and the solution was warmed to room temperature and stirred for an additional 30 minutes. The solvent was evaporated and the residue was dissolved in ethyl acetate. The organic layer was washed successively with saturated sodium bicarbonate solution, water, and brine, dried over $MgSO_4$, filtered, and concentrated to dryness. The crude product, mp 108—115°, was not purified further. Structure assignment was supported by nmr and infrared spectra.

IR (KBr): 1680 $cm^{-1}$.

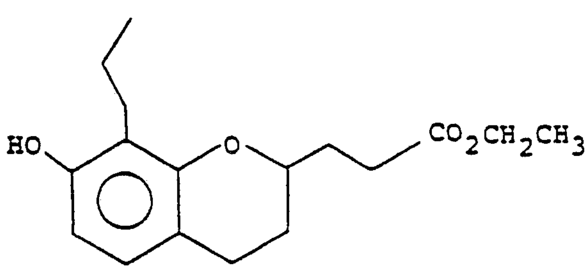

(A)

Example 11

ethyl 3-(3,4-dihydro-7-hydroxy-8-propyl-2H-1-benzopyran-2-yl)propanoate

A solution of 2.0 g (5.1 mmole) ethyl 3-(4-oxo-7-phenylmethoxy-8-propyl-4H-1-benzopyran-2-yl)-2-propenoate in 40 ml of acetic acid was hydrogenated at 50 psi and 70° over 5% palladium on carbon catalyst. The catalyst was removed by filtration and the filtrate was evaporated to dryness to give 1.3 g of the title compound as an oil. Structure assignment was supported by nmr and infrared spectra.

nmr ($CDCl_3$): δ (ppm) 0.95 (t, J=7Hz, 3H, propyl $CH_3$); 1.27 (t, J=7Hz, 3H, ester $CH_3$); 3.9 (m, 1H, 2-proton); 4.13 (q, J=7Hz, 2H, ester $CH_2$); 6.29, 6.69 (pair d, aromatic).

IR ($CHCl_3$): 1725 $cm^{-1}$.

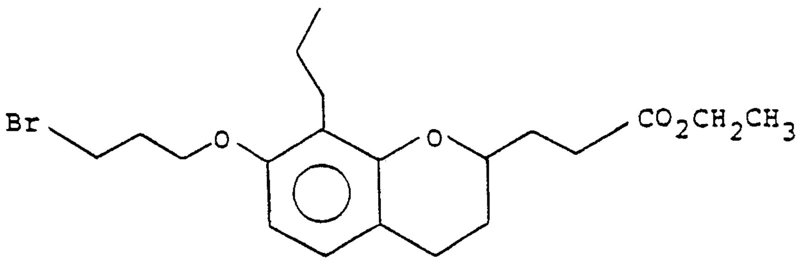

(A)

Example 12

ethyl 3-[7-(3-bromopropoxy)-3,4-dihydro-8-propyl-2H-1-benzopyran-2-yl]propanoate

The title compound was prepared by the method of Example 7 using the product of Example 11 as starting material. Structure assignment was supported by nmr and infrared spectrra.

nmr ($CDCl_3$): additional signals include δ (ppm) 3.59 (t, J=7Hz, 2H, $CH_2Br$) (compare Example 27).

IR ($CHCl_3$): 1725 $cm^{-1}$.

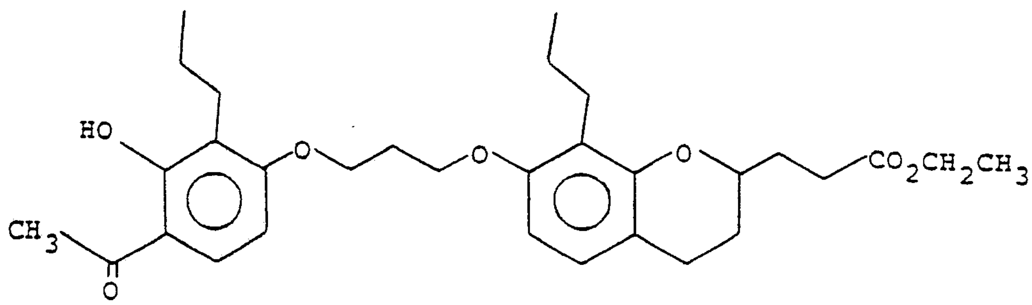

(B)

Example 13

ethyl 3-[7-[3-(4-acetyl-3-hydroxy-2-propylphenoxy)propoxy]-3,4-dihydro-8-propyl-2H-1-benzopyran-2-yl]propanoate

The title compound, mp 68—70°, was prepared by the method of Example 8 using the product of Example 12 as starting material. Structure assignment was confirmed by nmr and infrared spectra and by elemental analysis.

nmr ($CDCl_3$): δ (ppm) 0.89 (t, J=7Hz, 6H, propyl $CH_3$'s); 1.25 (t, J=7Hz, 3H, ester $CH_3$); 2.27 (t, J=6H, 2H, $CH_2C\underline{H},CH_2$); 2.54 (s, 3H, acetyl $CH_3$); 3.9 (m, 1H, 2-proton); 6.39, 6.42, 6.78, 7.54 (sets of d's, aromatic).

IR ($CHCl_3$): 1625, 1725 $cm^{-1}$.

| | | |
|---|---|---|
| Analysis. Calcd. for $C_{31}H_{42}O_7$: | C, 70.70; | H, 8.04. |
| Found: | C, 70.57; | H, 8.09. |

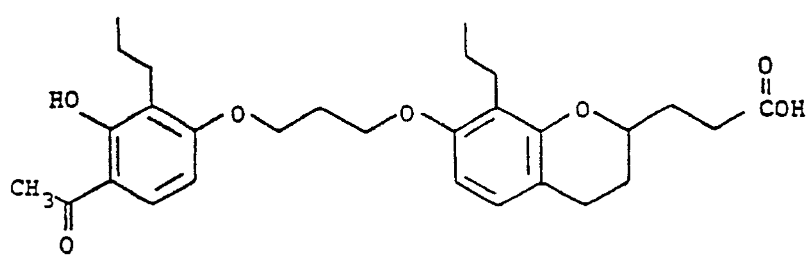

(B)

Example 14

3-[7-[3-(4-acetyl-3-hydroxy-2-propylphenoxy)propoxy]-3,4-dihydro-8-propyl-2H-1-benzopyran-2-yl]propanoic acid

The title compound, mp 109—110°, was prepared by the method of Example 9 using the product of Example 13 as starting material. Structure assignment was confirmed by nmr and infrared spectra and by elemental analysis.

nmr ($CDCl_3$): loss of ester protons (compare Example 13).

IR (KBr): 1635, 1705 $cm^{-1}$.

| | | |
|---|---|---|
| Analysis. Calcd. for $C_{29}H_{38}O_7$: | C, 69.86; | H, 7.68. |
| Found: | C, 69.83; | H, 7.68. |

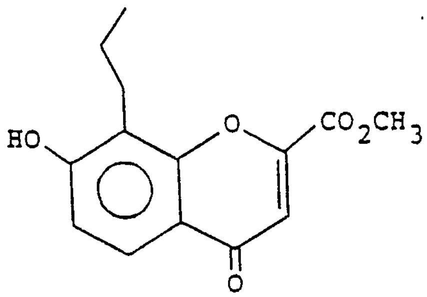

(A)

Example 15

methyl 7-hydroxy-4-oxo-8-propyl-4H-1-benzopyran-2-oate

To a solution of 99.7 g (0.513 mole) of 2,4-dihydroxy-3-propylacetophenone and 72.7 g (0.77 mole) of dimethyl oxalate in 1 liter of dry dimethylformamide was added in portions 97 g (1.8 mmole) of sodium methoxide. After stirring for 4 hours, 1.8 l of acetic acid was added and stirring was continued for 4 days. The reaction mixture was heated in a steam bath for four hours and evaporated to dryness. Water was added to the residue and the mixture was heated to boiling. A solid was filtered off and recrystallized from ethanol to give 89.8 g of the title compound, mp 222—224°. Structure assignment was supported by nmr and infrared spectra.

IR (KBr): 1650, 1745 $cm^{-1}$.

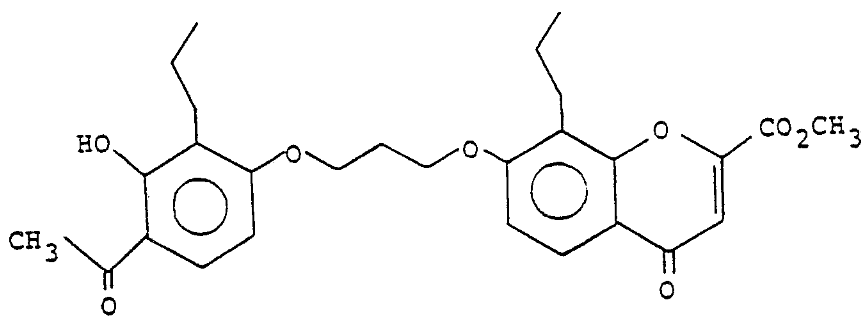

(A)

Example 16

methyl 7-[3-(4-acetyl-3-hydroxy-2-propyl phenoxy)propoxy]-4-oxo-8-propyl-4H-1-benzopyran-2-oate

The title compound was prepared by the method of Example 8 using the product of Example 15 as starting material. Structure assignment was confirmed by nmr and infrared spectra.

nmr ($CDCl_3$): δ (ppm) 0.89, 0.93 (pair t, 6H, propyl $CH_3$'s); 2.55 (s, 3H, acetyl $CH_3$); 2.62, 2.88 (pair t, 4, $OCH_2$'s); 6.98 (s, 1H, C=CH); 6.42, 7.00, 7.54, 8.00 (sets of d's, aromatic).

IR ($CHCl_3$): 1650, 1745 $cm^{-1}$.

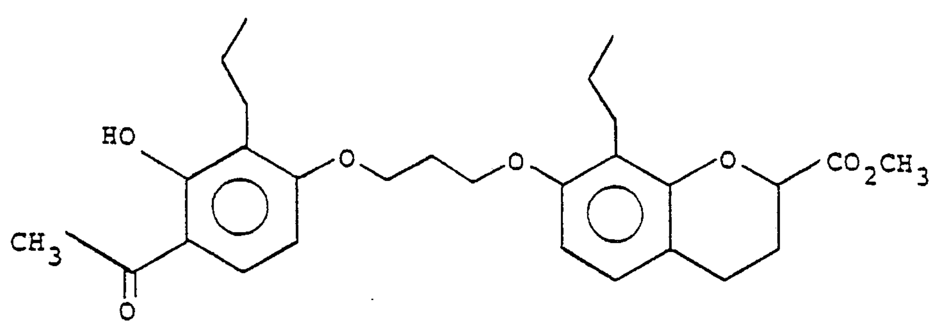

(A)

Example 17

methyl 7-[3-(4-acetyl-3-hydroxy-2-propyl phenoxy)propoxy]-3,4-dihydro-8-propyl-2H-1-benzopyran-2-oate

The title compound was prepared by the method of Example 11 using 30.7 g (61.9 mmole) of the product of Example 16 as starting material. Structure assignment was supported by nmr and infrared spectra.

nmr ($CDCl_3$): loss of alkenyl proton, addition of 2-proton at δ (ppm) 4.41 (t, J=5Hz) (compare Example 35).

IR ($CHCl_3$): 1610, 1620, 1730, 1750 $cm^{-1}$.

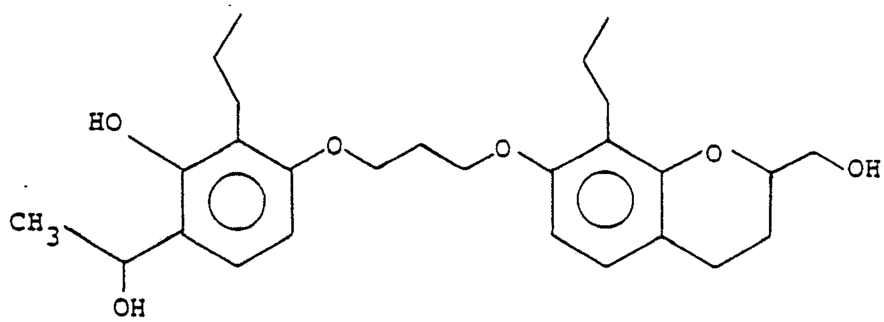

(B)

Example 18

7-[3-(3-hydroxy-4-(1-hydroxyethyl)-2-propylphenoxy)propoxy]-3,4-dihydro-8-propyl-2H-1-benzopyran-2-ylmethanol

A solution of 2.8g (0.13 mole) of lithium borohydride in 500ml of tetrahydrofuran was added to a solution of 25.0 g (51.6 mmole) of the title compound of Example 17 in 200 ml of tetrahydrofuran. The reaction mixture was stirred at room temperature for two hours and heated to reflux for one hour. After cooling the mixture, the excess borohydride reagent was destroyed by the addition of water and then a small amount of dilute hydrochloric acid. The reaction mixture was diluted with ethyl acetate and washed successively with sodium bicarbonate solution, water and brine, dried over $MgSO_4$, filtered, and evaporated to dryness. Chromatography of the residue on silica gel using 40% ethyl acetate/hexane as eluent gave 16.5 g of the title compound as an oil. Structure assignment was supported by nmr and infrared spectra.

nmr ($CDCl_3$): loss of ester $CH_3$, change in acetyl $CH_3$ to δ (ppm) 1.55 (d, J = 7Hz); additional signal at 4.96 (q, J = 6Hz, 1H, methyl-CHO—) (compare Example 17).

IR($CHCl_3$): no carbonyl band between 1600 and 1800 $cm^{-1}$.

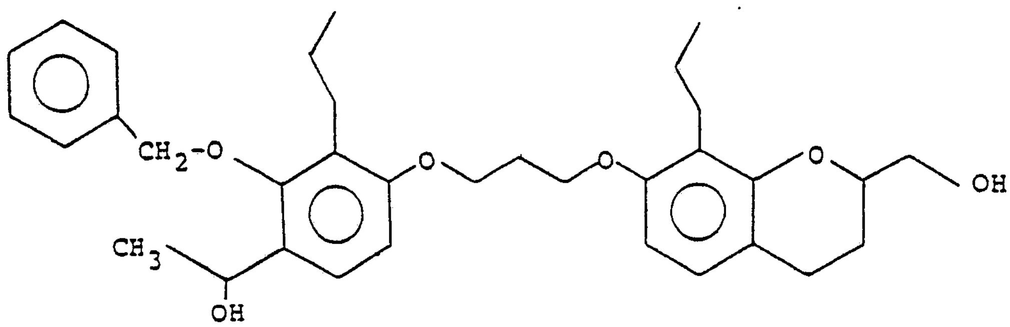

(A)

Example 19

7-[3-(4-(1-hydroxyethyl)-3-(phenylmethoxy)-2-propylphenoxy)propoxy]-3,4-dihydro-8-propyl-2H-1-benzopyran-2-ylmethanol

The title compound was prepared by the general method of Example 7 using the product of Example 18 and benzyl chloride as starting materials. Structure assignment was supported by nmr and infrared spectra.

IR($CHCl_3$): no carbonyl band between 1600 and 1800 $cm^{-1}$.

nmr ($CDCl_3$): additional aromatic protons (compare Example 18).

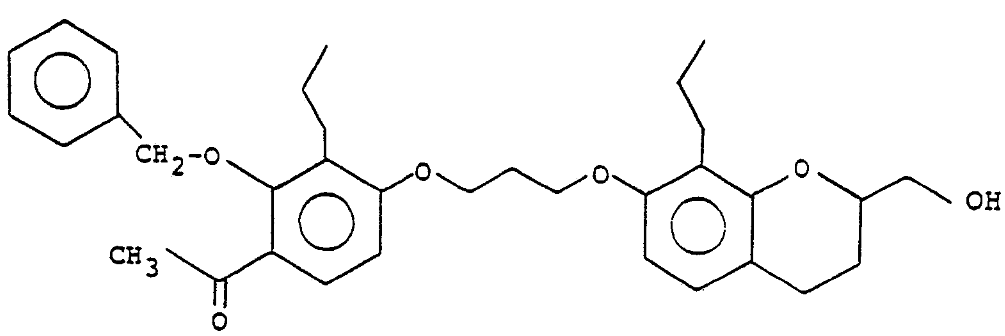

(A)

Example 20

7-[3-(4-acetyl-3-(phenylmethoxy)-2-propylphenoxy)propoxy]-3,4-dihdyro-8-propyl-2H-1-benzopyran-2-ylmethanol

To a solution of 8.9g (16.2 mmole) of the title compound of Example 19 in 150 ml of methylene chloride

was added 18 g (162 mmole) of manganese dioxide. The reaction mixture was stirred at room temperature for 12 hours and filtered. Removal of the solvent gave 6.9 g of the title compound, mp. 83—85°. Structure assignment was supported by nmr and infrared spectra.

IR($CHCl_3$): 1665 $cm^{-1}$.

nmr ($CDCl_3$): reappearance of acetyl $CH_3$ at δ(ppm) 2.55 (s, 3H) (compare Examples 17, 18, 19).

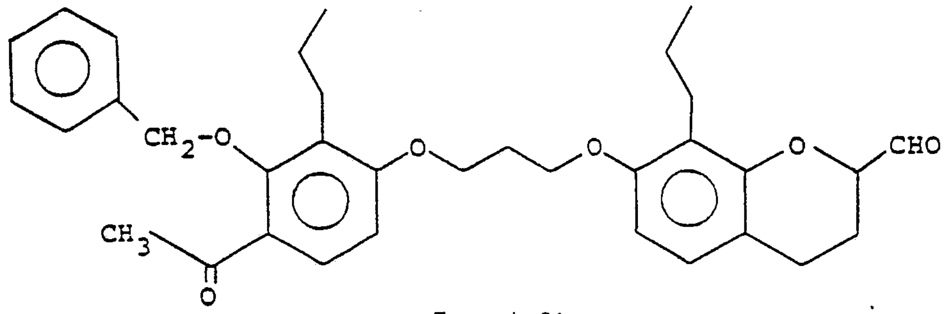

(A)

Example 21

7-[3-(4-acetyl-3-(phenylmethoxy)-2-propylphenoxy)propoxy]-3,4-dihydro-8-propyl-2H-1-benzopyran-2-carboxaldehyde

A solution of 1.2 ml (13.3 mmole) of oxalyl chloride in 30 ml of methylene chloride was cooled to −70°, and a solution of 1.9 ml (26.6 mmole) of dimethylsulfoxide in 9 ml of methylene chloride was added. The mixture was stirred for 5 minutes and a solution of 6.6 g (12.1 mmole) of the title compound of Example 20 in 20 ml of methylene chloride was added. After stirring an additional 15 minutes, 8.5 ml (60.5 mmole) of triethylamine was added, and the mixture was stirred at −70° for 5 minutes and allowed to warm to room temperature. Water was added and the layers were separated. The organic layer was washed successively with dilute hydrochloric acid, 5% sodium bicarbonate solution, water and brine, dried over $MgSO_4$, and filtered.

Removal of the solvent gave 6.44 g of the title compound as a yellow oil. Structure assignment was supported by nmr and infrared spectra.

nmr ($CDCl_3$): δ(ppm) 9.76 (s, 1H, CHO)

IR($CHCl_3$): 1665, 1735 $cm^{-1}$.

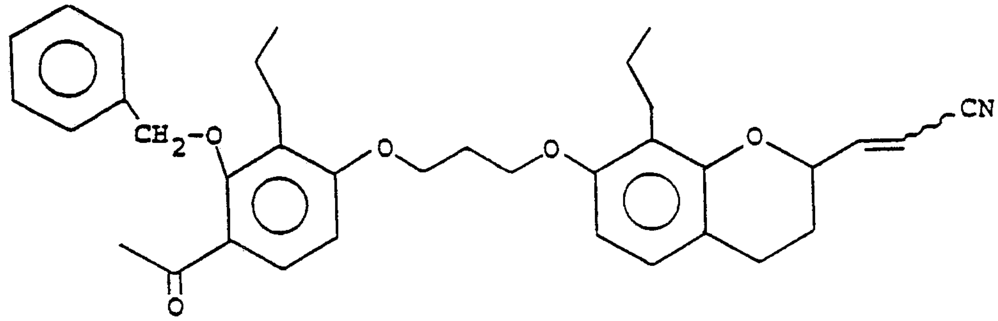

(A)

Example 22

3-[7-[3-(4-acetyl-3-(phenylmethoxy)-2-propylphenoxy)propoxy]-3,4-dihydro-8-propyl-2H-1-benzopyran-2-yl]-2(E and Z)-propenenitrile

To a solution of 6.35 (11.7 mmole) of the title compound of Example 21 in 150 ml of benzene was added 4.04 g (13.5 mmole) of cyanomethylenetriphenylphosphorane. The reaction mixture was stirred for 12 hours at room temperature and evaporated to dryness. Chromatography of the residue on silica gel using 20% ethyl acetate/hexane as eluent gave 1.3 g of the cis isomer, 0.8 g of the trans isomer and 1.1g of a 53:47 cis/trans mixture. Structure assignment was supported by nmr and infrared spectra.

nmr ($CDCl_3$) (cis isomer): δ(ppm) 5.44 (dd, $J_1$ = 11Hz, $J_2$ = 1.5Hz, 1H, CHCN) (trans isomer): δ(ppm) 5.69 (dd, $J_1$ = 16Hz, $J_2$ = 3Hz 1H, CHCN); 6.77 (dd, $J_1$ = 16Hz, $J_3$ = 4Hz, 1H, HC = C—CN)

IR:$CHCl_3$) (cis isomer): 1665, 2210 $cm^{-1}$ (trans isomer): 1665, 2230 $cm^{-1}$.

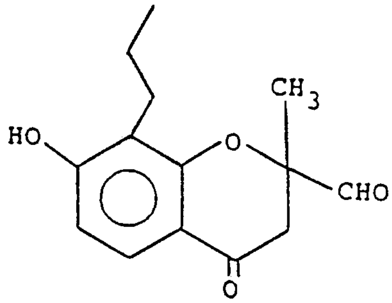

(A)

Example 23

3,4-dihydro-7-hydroxy-2-methyl-4-oxo-8-propyl-2H-2-benzopyran-2-carboxaldehyde

A solution of 30.0 g of 2,4-dihydroxy-3-propylacetophenone in 125 ml of toluene containing 15.5ml of pyrrolidine was heated at reflux using a Dean-Stark trap to remove water. After 29.2 ml of pyruvaldehyde dimethyl acetal was added to the cooled (*ca.* 0°) solution, the mixture was heated to reflux as additional water was removed. After five hours an additional 5.2 ml of pyrrolidine was added and the mixture was refluxed a further 18 hours. The reaction mixture was cooled, concentrated, and taken up in ethyl acetate. The resultant solution was washed sequentially with water, 1 *N* hydrochloric acid, and brine, dried over magnesium sulfate, filtered, and concentrated to dryness. The residue was dissolved in ethyl acetate/hexane, filtered through silica gel, and concentrated to an oil which was purified by column chromatography on silica gel. The intermediate acetal derivative of the title compound was collected as a yellow solid (13.7 g) which was used without further purification to prepare the title compound. (An analytically pure sample, m.p. 146—148°, was recrystallized from ethyl acetate/hexane.) A solution of 2.0 g of the intermediate acetal in 20 ml of acetic acid containing 10 ml of 10% sulfuric acid was heated at 80° for three hours. The mixture was taken up in ethyl acetate, washed with water, aqueous sodium bicarbonate, and brine, then dried with magnesium sulfate, filtered, and concentrated. The title compound, isolated as 1.7 g of an oil, was used in subsequent reactions without further purification. Structure assignment was supported by nmr and infrared spectra.

nmr ($CDCl_3$): δ (ppm) 0.99, 1.55, 2.70 (t, m, t, 7H, propyl protons); 1.53 (s, 3H, methyl); 2.88 (g, 2H, ring $CH_2$); 6.52, 7.62 (pair d, aromatic); 9.60 (s, 1H, CHO)

IR ($CHCl_3$): 1740, 1675 $cm^{-1}$.

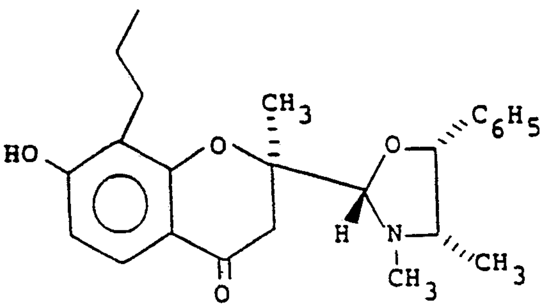

(A)

Example 24

2R-(3,4S-dimethyl-5R-phenyl-2S-oxazolidinyl)-3,4-dihydro-7-hydroxy-2-methyl-8-propyl-2H-1-benzopyran-4-one

A solution of 20.0 g of the title compound of Example 23 and excess 1-ephedrine in 150 ml of benzene was heated at reflux using a Dean-Stark trap to remove water. After four hours at reflux the solution was filtered through silica gel, washing with 50% by volume ethyl acetate/hexane, and then concentrated to dryness *in vacuo.* Column chromatography on silica gel using ethyl acetate/hexane, after recycling to obtain only the least polar component, afforded 1.1 g of the title compound, m.p. 196—197°. Structure determination was confirmed by X-ray crystallography.

$[\alpha]_D$ −2.1° (1.029% in acetone).

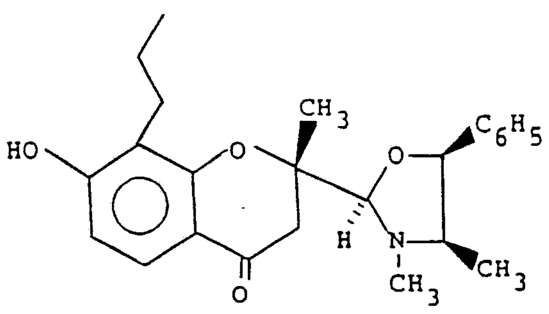

(A)

Example 25

2S-(3,4R-dimethyl-5S-phenyl-2R-oxazolidinyl)-3,4-dihydro-7-hydroxy-2-methyl-8-propyl-2H-1-benzopyran-4-one

The title compound (4.85 g), m.p. 196—198°, was prepared by the method of Example 24 using 17 g of the title compound of Example 23 and excess d-ephedrine. The optical rotation of the title compound was opposite in sign to that for the title product of Example 24.

$[\alpha]_D$ +1.6° (1.014% in acetone).

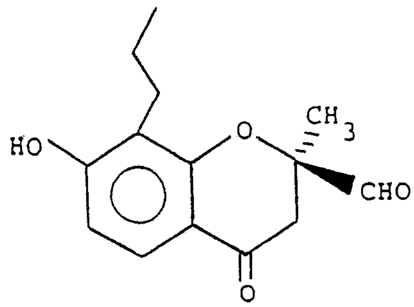

(A)

Example 26

3,4-dihydro-7-hydroxy-2-methyl-4-oxo-8-propyl-2H-1-benzopyran-2R-carboxaldehyde

The title compound of Example 24 (4.1 g) was dissolved in 150 ml of methanol to which was then added 12 g of Dowex®-50 ($H^+$ form) and 50 ml of water. After 2.5 hours at 50° the mixture was filtered and the filtrate concentrated *in vacuo* to give 1.2 g of the title compound. A second crop of the product was obtained by suspending the recovered exchange resin in 100 ml of 90% aqueous methanol, adding an additional 4 g of Dowex-50, heating at 60° for three hours, filtering, and concentrating to dryness. A total of 2.4 g of aldehyde product was obtained. The (R)-aldehyde was used for subsequent reactions without further purification.

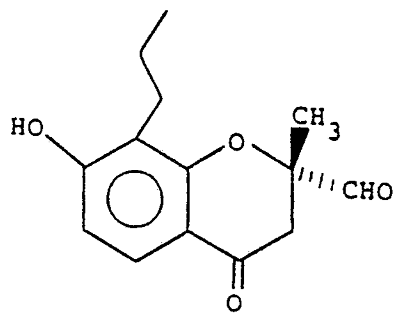

(A)

Example 27

3,4-dihydro-7-hydroxy-2-methyl-4-oxo-8-propyl-2H-1-benzopyran-2S-carboxaldehyde

The title compound (3.05 g) was prepared by the method of Example 26 using 5.0 g of the title compound of Example 25. The (S)-aldehyde was used for subsequent reactions without further purification.

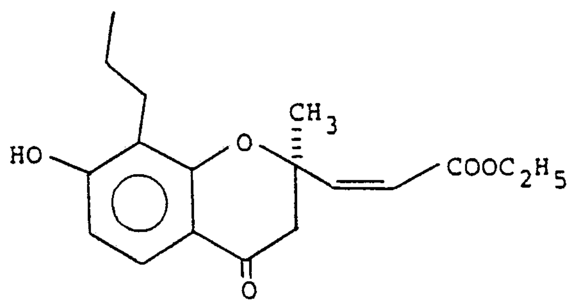

(A)

Example 28

ethyl 3-(3,4-dihydro-7-hydroxy-2R-methyl-4-oxo-8-propyl-2H-1-benzopyran-2-yl)-2(Z)-propenoate

A solution of 1.2 g of the (R)-aldehyde product of Example 26 and 1.8 g of (carbethoxymethylene)-triphenylphosphorane was allowed to stand for about seven hours. The solution was filtered through silica gel and the filtrate concentrated to dryness. The residue was then chromatographed on silica gel using 18% ethyl acetate/hexane as eluent. Early chromatographic fractions afforded the title compound (i.e., the cis isomer), m.p. 104—106°.

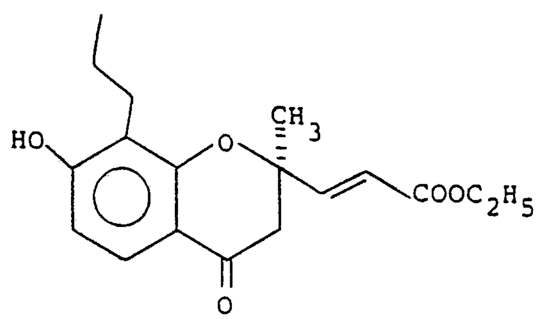

(A)

Example 29

ethyl 3-(3,4-dihdyro-7-hydroxy-2R-methyl-4-oxo-8-propyl-2H-1-benzopyran-2-yl)-2(E)-propenoate

Later chromatographic fractions of Example 28 afforded the trans isomer, m.p. 111.5—112.5°. Structure assignment was supported by nmr spectra, optical rotation, and elemental analysis.

nmr ($CDCl_3$): alkenyl protons at δ (ppm) 6.00 (d, J = 16 Hz), 6.93 (d, J = 16Hz)

$[\alpha]_D$ +129.0° (0.903% in $CHCl_3$)

| | | |
|---|---|---|
| Analysis Calculated for $C_{18}H_{22}O_5 \cdot 1/2H_2O$: | C, 66.96; | H, 7.02. |
| Found: | C, 67.08; | H, 6.95 |

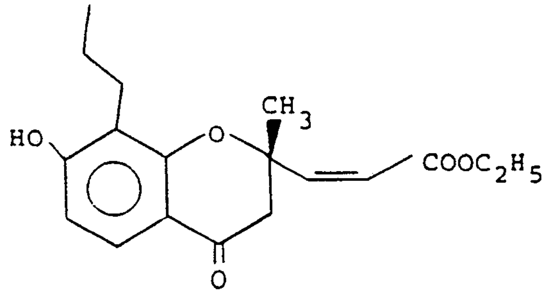

(A)

Example 30

ethyl 3-(3,4-dihydro-7-hydroxy-2S-methyl-4-oxo-8-propyl-2H-1-benzopyran-2-yl)-2(Z)-propenoate

The title compound (i.e., the cis isomer), m.p. 109—110°, was prepared by the method of Example 28 using 3.05 g of the (S)-aldehyde product of Example 27 and 4.7 g of the phosphorane reagent. Structure assignment was supported by optical rotation and elemental analysis.

$[\alpha]_D$ −185.8° (0.771% in $CHCl_3$).

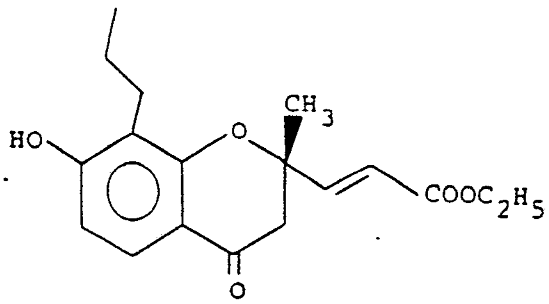

(A)

Example 31

ethyl 3-(3,4-dihdyro-7-hydroxy-2S-methyl-4-oxo-8-propyl-2H-1-benzopyran-2-yl)-2(E)-propenoate

Later chromatographic fractions of Example 30 afforded the trans isomer, m.p. 110.5—111.5°. Structure assignment was supported by optical rotation, and elemental analysis.

$[\alpha]_D$ −125.7° (1.015% in $CHCl_3$)

| | | |
|---|---|---|
| Analysis Calculated for $C_{18}H_{22}O_5$: | C, 67.91; | H, 6.97. |
| Found: | C, 67.67; | H, 6.98 |

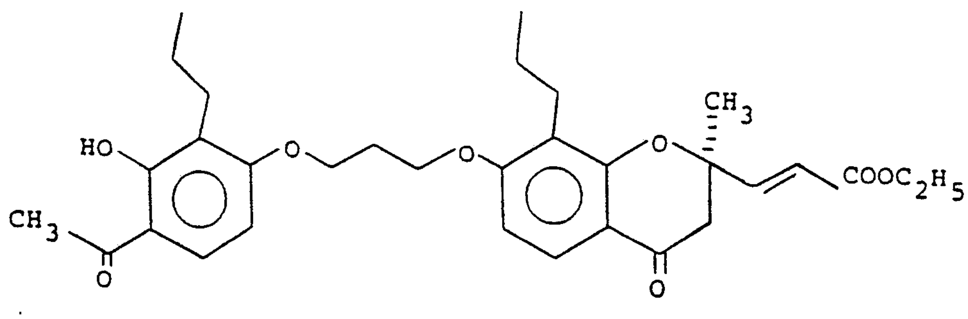

(A)

Example 32

ethyl 3-[7-[3-(4-acetyl-3-hydroxy-2-propylphenoxy)propoxy)-3,4-dihydro-2R-methyl-4-oxo-8-propyl-2H-1-benzopyran-2-yl)-2(E)-propenoate

A mixture of 1.1 g of the title compound of Example 29, 1.0 g of the title compound of Example 5, and 0.91 g of powdered potassium carbonate in 30 ml of dimethylformamide was stirred at room temperature overnight. The reaction mixture was diluted with ethyl acetate and filtered, and the filtrate was washed sequentially with ammonium chloride solution and brine, filtered and concentrated *in vacuo* to an oil. Column chromatography on silica gel using 7% ethyl acetate/toluene afforded 1.2 g of the title compound, m.p. 135.5—136.5°. Structure assignment was supported by optical rotation and elemental analysis.

[α]$_D$ +91.8° (1.000% in $CHCl_3$)

| | | |
|---|---|---|
| Analysis Calculated for $C_{32}H_{40}O_5$: | C, 69.54; | H, 7.30. |
| Found: | C, 69.32; | H, 7.25 |

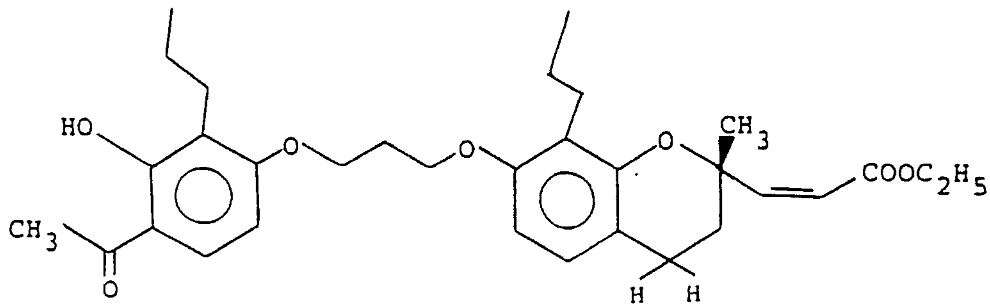

(B)

Example 33

ethyl 3-[7-[3-(4-acetyl-3-hydroxy-2-propylphenoxy)propoxy]-3,4-dihydro-2S-methyl-4-oxo-8-propyl-2H-1-benzopyran-2-yl)-2(Z)-propenoate

The title compound m.p. 98—99°, was prepared by the method of Example 32 using the title compound of Example 30. Structure assignment was supported by optical rotation and elemental analysis.

[α]$_D$ −118.8° (1.013% in $CHCl_3$)

| | | |
|---|---|---|
| Analysis Calculated for $C_{32}H_{40}O_8$: | C, 69.54; | H, 7.30. |
| Found: | C, 69.63; | H, 7.30 |

CHART A

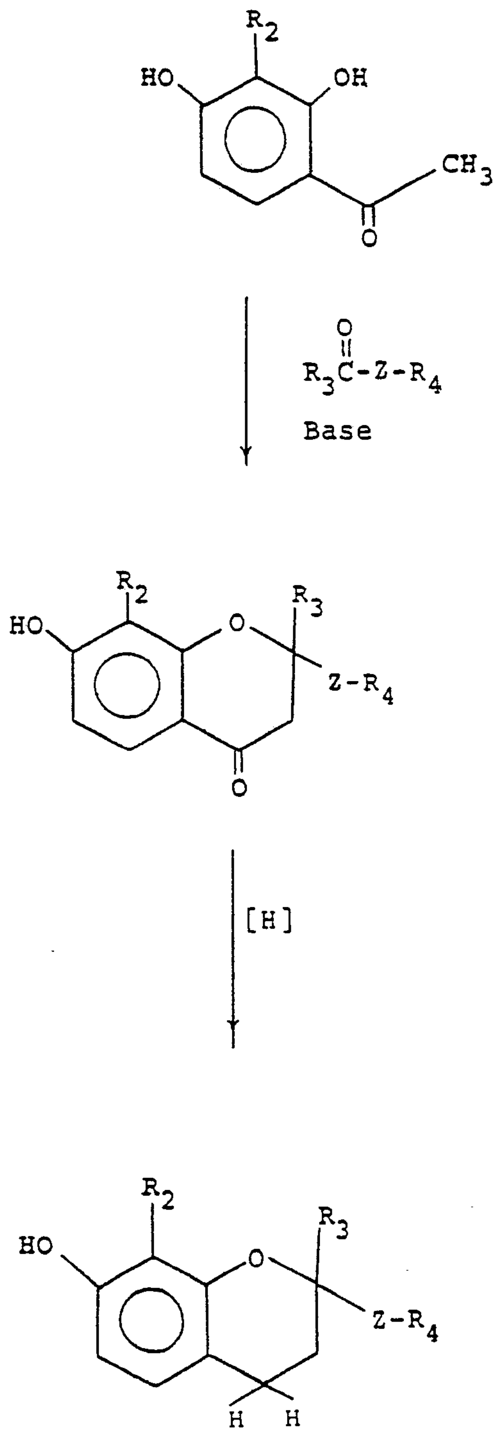


XI

XII

XIII

XIV

CHART B

XIV

Base

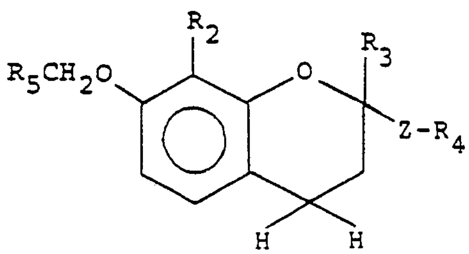

XXI

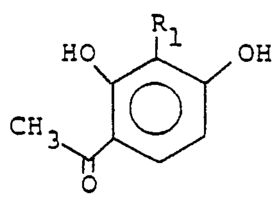

XXII

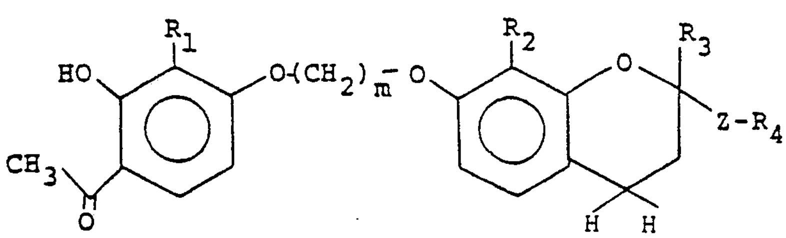

XXIII

CHART C

XXII

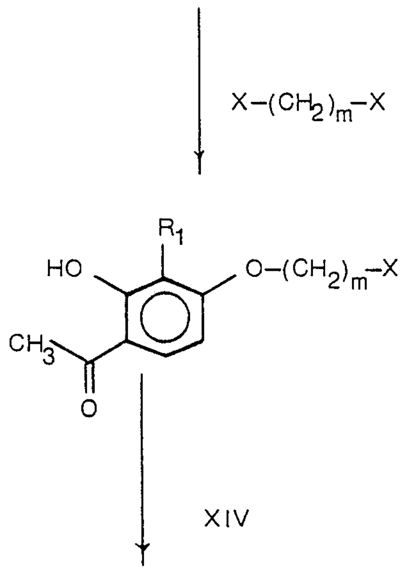


XXXI

XXIII

CHART D

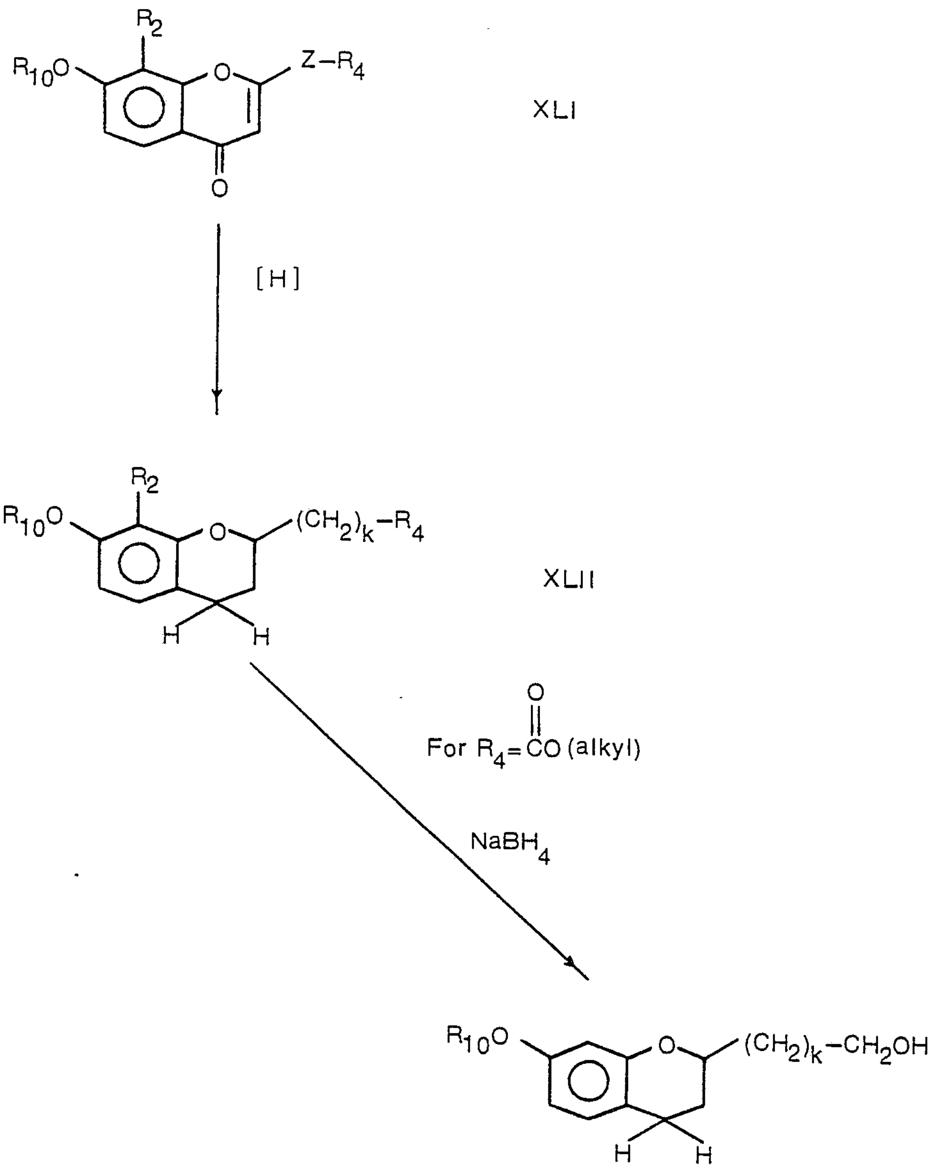


XLI

XLII

XLIV

CHART E

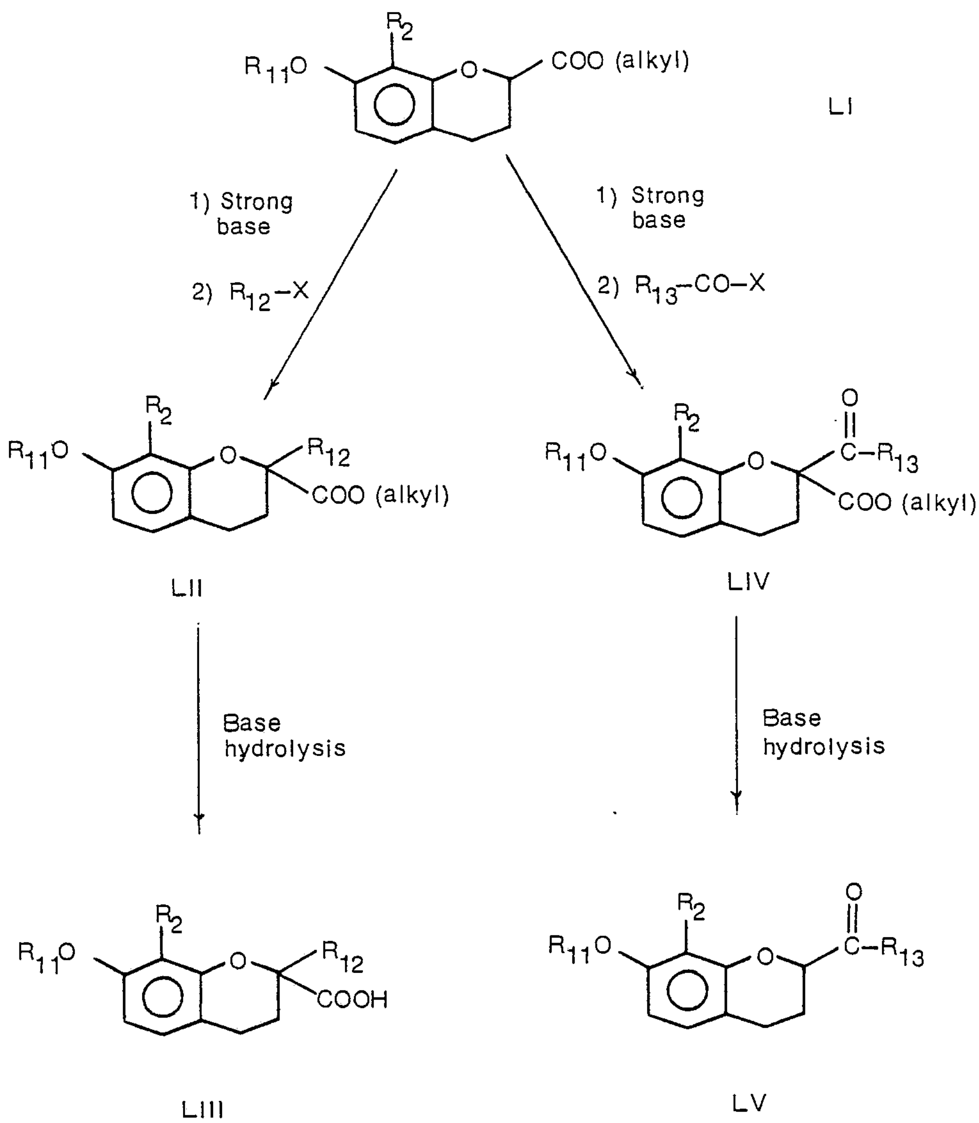


LI

LII

LIV

LIII

LV

## Claims

1. A compound of the formula

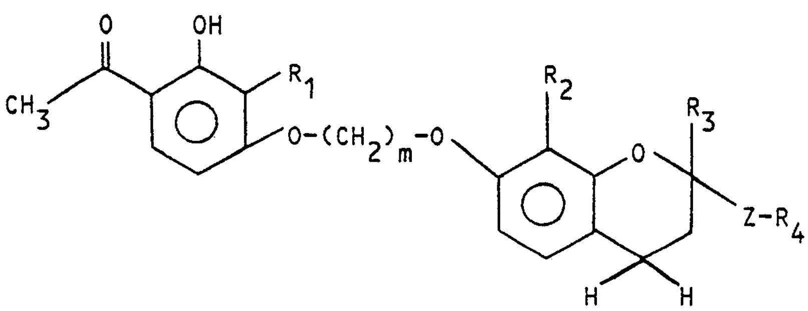

wherein Z is:

a) $-(CH_2)_n-$; or

b) $-(CH_2)_p-CH=CH-(CH_2)_q-$;

wherein m is an integer of from 2 to 6 inclusive;

wherein n is an integer of from 1 to 3 inclusive;
wherein p is an integer of from zero to 4 inclusive;
wherein q is an integer of from zero to 4 inclusive;
wherein p+q is equal to or less than 6;
wherein $R_1$, $R_2$ and $R_6$ are:
a) alkyl of 1 to 6 carbon atoms, inclusive, each being the same or different:
wherein $R_3$ is:
a) hydrogen; or
b) alkyl of 1 to 6 carbon atoms, inclusive;
wherein $R_4$ is:
a) $—CO_2H$;
b) $—CO_2R_6$;
c) $—CONR_7R_8$; or
d) —OH
wherein $R_7$ and $R_8$ are:
a) hydrogen;
b) alkyl of 1 to 6 carbon atoms, inclusive; $R_7$ and $R_8$ each being the same or different; or
c) taken together with the nitrogen atom to which they are attached form a 5 or 6 member saturated N-heterocycle optionally substituted by carboxylic acid or alkyl esters thereof of 1 to 6 carbon atoms, inclusive, with the balance of the members being carbon;
or the pharmacologically acceptable addition salts thereof.

2. A compound according to Claim 1 wherein $R_3$ is alkyl.

3. Methyl 3 - [1 - [3 - (4 - acetyl - 3 - hydroxy - 2 - propylphenoxy)propoxy] - 3,4 - dihydro - 2 - methyl - 8 - propyl - 2H - 1 - benzopyran - 2 - yl]propanoate, a compound according to Claim 2.

4. 3 - [1 - [3 - (4 - acetyl - 3 - hydroxy - 2 - propylphenoxy)propoxy] - 3,4 - dihydro - 2 - methyl - 8 - propyl - 2H - 1 - benzopyran - 2 - yl]propanoic acid, a compound according to Claim 2.

5. A compound according to Claim 1 wherein $R_3$ is —H.

6. Ethyl 3 - [1 - [3 - (4 - acetyl - 3 - hydroxy - 2 - propylphenoxy)propoxy] - 3,4 - dihydro - 8 - propyl - 2H - 1 - benzopyran - 2 - yl]propanoate, a compound according to Claim 5.

7. 3 - [1 - [3 - (4 - acetyl - 3 - hydroxy - 2 - propylphenoxy)propoxy] - 3,4 - dihydro - 8 - propyl - 2H - 1 - benzopyran - 2 - yl]propanoic acid, a compound according to Claim 5.

**Patentansprüche**

1. Eine Verbindung der Formel:

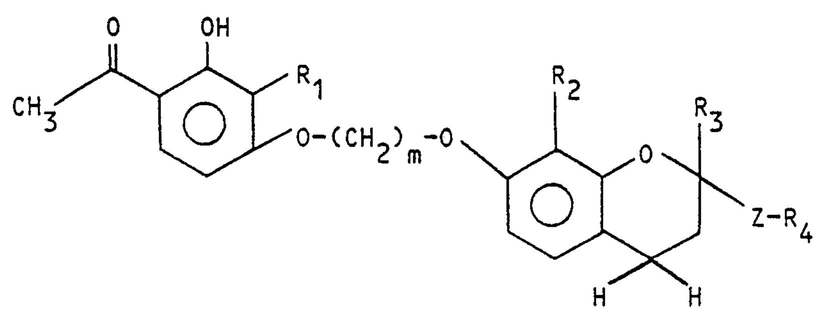

worin Z
a) $—(CH_2)_n—$ oder
b) $—(CH_2)_p—CH= CH—(CH_2)_q—$; ist
worin
m eine Zahl von 2 bis 6 einschliesslich,
n eine Zahl von 1 bis 3 einschliesslich,
p eine Zahl von 0 bis 4 einschliesslich,
q eine Zahl von 0 bis 4 einschliesslich und
worin p+q gleich oder weniger als 6 ist,
und worin $R_1$, $R_2$ und $R_6$ gleich oder verschieden sind
und
a) Alkylgruppen mit 1 bis 6 C-Atomen einschliesslich bedeuten,
$R_3$
a) Wasserstoff oder
b) ein Alkylrest mit 1 bis einschliesslich 6 C-Atomen ist,
$R_4$
a) —COOH
b) $—CO_2R_6$

c) $-CONR_7R_8$ oder
d) $-OH$
ist
und worin $R_7$ und $R_8$
a) Wasserstoff
b) eine Alkylgruppe mit 1 bis einschliesslich 6 C-Atomen bedeuten und gleich oder verschieden sind oder
c) $R_7$ und $R_8$ zusammen mit dem Stickstoffatom, an das sie gebunden sind, einen 5- oder 6-gliedrigen gesättigten N-Heterocyclus bilden, welcher wahlweise substituiert sein kann durch eine Carbonsäure oder deren Alkylester mit 1 bis einschliesslich 6 Kohlenstoffatomen, wobei die anderen Ringglieder Kohlenstoffatome sind,
oder pharmakologisch akzeptable Säure-Additionssalze hiervon.

2. Eine Verbindung gemäss Anspruch 1, worin $R_3$ Alkyl bedeutet.

3. 3 - [7 - [3 - (4 - Acetyl - 3 - hydroxy - 2 - propylphenoxy)propoxy] - 3,4 - dihydro - 2 - methyl - 8 - propyl - 2H - 1 - benzopyran - 2 - propionsäuremethylester, eine Verbindung gemäss Anspruch 2.

4. 3 - [7 - [3 - (4 - Acetyl - 3 - hydroxy - 2 - propylphenoxy)propoxy] - 3,4 - dihydro - 2 - methyl - 8 - propyl - 2H - 1 - benzopyran - 2 - propionsäure, eine Verbindung gemäss Anspruch 2.

5. Eine Verbindung gemäss Anspruch 1, worin $R_3$ H bedeutet.

6. 3 - [7 - [3 - (4 - Acetyl - 3 - hydroxy - 2 - propylphenoxy)propoxy] - 3,4 - dihydro - 8 - propyl - 2H - 1 - benzopyran - 2 - propionsäureethylester, eine Verbindung gemäss Anspruch 5.

7. 3 - [7 - [3 - (4 - Acetyl - 3 - hydroxy - 2 - propylphenoxy)propoxy) - 3,4 - dihydro - 8 - propyl - 2H - 1 - benzopyran - 2 - propionsäure, eine Verbindung gemäss Anspruch 5.

**Revendications**

1. Un composé de formule;

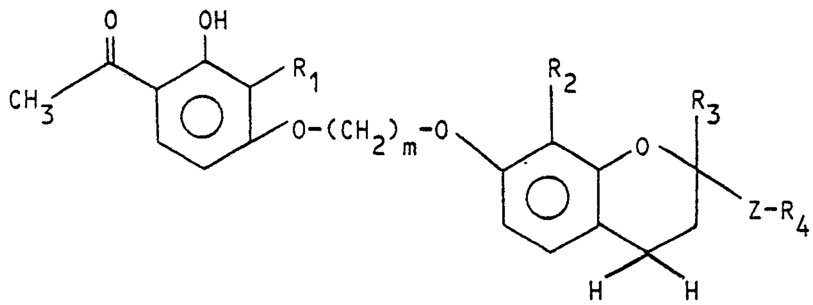

dans laquelle Z est:
a) $-(CH_2)_n-$; ou
b) $-(CH_2)_p-CH=CH-(CH_2)_q-$;
m est un entier de 2 à 6 inclus;
n est un entier de 1 à 3 inclus;
p est un entier de 0 à 4 inclus;
q est un entier de 0 à 4 inclus;
où la somme p+q est égal ou inférieure à 6;
dans laquelle $R_1$, $R_2$ et $R_3$ sont identiques ou différents et représentent chacun
a) un alkyle en $C_1-C_6$ inclus;
$R_3$ est:
a) l'hydrogène; ou
b) un alkyle en $C_1-C_6$ inclus;
$R_4$ est:
a) $-CO_2H$;
b) $-CO_2R_6$;
c) $-CONR_7R_8$; ou
d) $-OH$
$R_7$ et $R_8$ sont identiques ou différents et représentent chacun
a) l'hydrogène;
b) un alkyle en $C_1-C_6$ inclus; ou bien
c) $R_7$ et $R_8$ pris ensemble avec l'atome d'azote auquel ils sont liés forment un hétérocycle azoté saturé à 5 ou 6 chaînons facultativement substitué par un acide carboxylique ou ses esters d'alkyles en $C_1-C_6$ inclus, le reste des chaînons étant du carbone;
ou ses sels acceptables en pharmacie.

2. Un composé selon la revendication 1, dans lequel $R_3$ est un alkyle.

3. Le 3 - [7 - [3 - (4 - acétyl - 3 - hydroxy - 2 - propylphénoxy) - propoxy] - 3,4 - dihydro - 2 - méthyl - 8 - propyl - 2H - 1 - benzopyranne - 2 - yl]propanoate de méthyle, composé selon la revendication 2.

4. L'acide 3 - [7 - [3 - (4 - acétyl - 3 - hydroxy - 2 - propylphénoxy)propoxy] - 3,4 - dihydro - 2 - méthyl - 8 - propyl - 2H - 1 - benzopyranne - 2 - yl]propanoïque, composé selon la revendication 2.

5. Un composé selon la revendication 1, dans lequel $R_3$ est —H.

6. Le 3 - [7 - [3 - (4 - acétyl - 3 - hydroxy - 2 - propylphénoxy)propoxy] - 3,4 - dihydro - 8 - propyl - 2H - 1 - benzopyranne - 2 - yl]propanoate d'ethyle, composé selon la revendication 5.

7. L'acide 3 - [7 - [3 - (4 - acétyl - 3 - hydroxy - 2 - propylphénoxy)propoxy] - 3,4 - dihydro - 8 - propyl - 2H - 1 - benzopyranne - 2 - yl]propanoïque, composé selon la revendication 5.